# EUROPÄISCHE PATENTSCHRIFT

(11) **EP 0 345 464 B1**
(45) Veröffentlichungstag und Bekanntmachung des Hinweises auf die Patenterteilung: **17.03.1993**
(21) Anmeldenummer: 89108006.1
(22) Anmeldetag: 03.05.1989
(51) Int. Cl.: C07C 229/34, C07C 227/04

(54) **Verfahren zur Herstellung von substituierten 3-Amino-2(benzoyl)-acrylsäureestern sowie ein Verfahren zur Herstellung von Zwischenprodukten für antibakterielle Wirkstoffe aus diesen Verbindungen**
Process for the preparation of substituted 3-amino-2-(benzoyl)acrylic acid esters as well as a process for the preparation of intermediates for antibacterial active agents from these compounds
Procédé pour la préparation d'esters d'acide 3-amino-2-(benzoyl) acrylique substitués ainsi qu'un procédé de préparation d'intermédiaires pour des constituants actifs antibactériens à partir de ces composés

(30) Priorität: 09.06.1988 AT 1495/88
(43) Veröffentlichungstag der Anmeldung: 13.12.1989
(73) Patentinhaber: Chemie Linz Gesellschaft m.b.H., A-4021 Linz (AT)
(72) Erfinder: Baumann, Karl, Dr., A-1190 Wien (AT); Fitzinger, Klaus, A-4240 Freistadt (AT)
(74) Vertreter: Kunz, Ekkehard, Dr.

(56) Entgegenhaltungen:
- EP-A- 0 088 299
- EP-A- 0 245 690
- CHEMICAL ABSTRACTS, Band 101, Nr. 7, 13. August 1984, Seite 564, Zusammenfassung Nr. 54435t, Colulmbus, Ohio, US; Z. BANKOWSKA et al, "Transmission of the effect of substituents in para-substituted ethyl alpha-cyanobenzoylacetate derivatives"
- BEILSTEINS HANDBUCH DER ORGANISCHEN CHEMIE, 4. Auflage, 3. Band, 1921, Springer-Verlag, Berlin, DE

## Beschreibung

Die Erfindung betrifft ein neues Verfahren zur Herstellung von substituierten 3-Amino-2-(benzoyl)-acrylsäureestern, sowie ein Verfahren zur Herstellung von Zwischenprodukten für antibakterielle Wirkstoffe aus diesen Verbindungen.

Aus DE-OS 36 15 767 sind 3-Aminoacrylsäureester als Ausgangsprodukte für die Herstellung von 4-Hydroxy-3-chinolincarbonsäuren bekannt. Diese werden aus substituierten 2-Benzoyl-3-alkoxy-acrylsäureestern durch Austauschen der 3-alkoxygruppe gegen ein geeignetes Amin erhalten. Die substituierten 2-Benzoyl-3-alkoxy-acrylsäureester werden durch Acylierung von Monoester mit subst. Benzoylhalogeniden zu den entsprechenden Acylmalonestern, partielle saure Verseifung und Decarboxylierung zu substituierten Benzoylessigsäureestern und anschließende Kondensation mit Orthoameisensäureester/Acetanhydrid erhalten. Dieses Verfahren umfaßt 4 Reaktionsstufen. Bei der partiellen Verseifung und Decarboxylierung der Acylester besteht darüberhinaus die Gefahr, daß Nebenprodukte entstehen.

Ziel der Erfindung war daher die Bereitstellung eines einfachen Verfahrens, das unter milden Reaktionsbedingungen mit ausgezeichneten Ausbeuten zu substituierten 3-Amino-2-(benzoyl)-acrylsäureestern führt, die begehrte Zwischenprodukte für die Herstellung antibakterieller Wirkstoffe darstellen.

Gegenstand der Erfindung ist daher ein Verfahren zur Herstellung von substituierten 3-Amino-2-(benzoyl)-acrylsäureestern der allgemeinen Formel I des Formelblattes, in der R₁ einen Alkylrest mit 1 bis 4 C-Atomen, X₁ Halogen, insbesondere Fluor oder Chlor, X₂, X₃, X₄, X₅ Wasserstoff, Halogen, insbesondere Fluor oder Chlor, einen Alkylrest mit 1 - 4 C-Atomen oder einen Alkoxyrest mit 1 - 4 C-Atomen in der Alkylkette bedeuten, das dadurch gekennzeichnet ist, daß man einen entsprechenden substituierten 2-Cyano-3-hydroxy-3(phenyl)-acrylsäureester der allgemeinen Formel II des Formelblattes in der R₁, X₁, X₂, X₃, X₄, X₅ die oben angegebene Bedeutung haben, selektiv reduziert.

In den Formeln I und II bedeutet R₁ einen geradkettigen oder verzweigten Alkylrest mit 1 - 4 C-Atomen, beispielsweise einen Methyl-, einen Ethyl-, einen Propyl-, einen i-Propyl, einen Butyl-, einen i-Butyl-, oder einen t-Butylrest.
X₁ bedeutet Halogen, insbesondere Fluor oder Chlor.
X₂, X₃, X₄ und X₅ können unabhängig voneinander Wasserstoff, Halogen, insbesondere Fluor oder Chlor bedeuten.

Weiters können die Substituenten X₂, X₃, X₄ und X₅ einen geradkettigen oder verzweigten Alkylrest mit 1 - 4 C-Atomen, beispielsweise einen Methyl-, einen Ethyl-, einen Propyl-, einen i-Propyl-, einen Butyl-, einen i-Butyl- oder einen t-Butylrest, oder einen geradkettigen oder verzweigten Alkoxyrest mit 1 - 4 C-Atomen in der Alkylkette, beispielsweise einen Methoxy-, einen Ethoxy-, einen Propoxy- einen Butoxyrest bedeuten. Vorzugsweise bedeuten X₃ und X₄ unabhängig voneinander Halogen, insbesondere Fluor oder Chlor. X₂ und X₅ bedeuten vorzugsweise Wasserstoff, Halogen, insbesondere Fluor oder Chlor oder einen geradkettigen oder verzweigten Alkylrest mit 1 - 4 C-Atomen.

Die als Ausgangsverbindungen für die substituierten 3-Amino-2-(benzoyl)-acrylsäureester der allgemeinen Formel I verwendeten substituierten 2-Cyano-3-hydroxy-3-(phenyl)acrylsäureester der allgemeinen Formel II können in an sich bekannter Weise aus den entsprechenden Benzoylhalogeniden, insbesondere den Benzoylchloriden durch Kondensation mit Cyanessigsäureestern hergestellt werden.
Dabei wird das entsprechende Benzoylhalogenid mit einem Cyanessigsäureester in Gegenwart einer Base, beispielsweise Natriumhydrid, Magnesiumalkoholat, Natriumalkoholat in einem wasserfreien organischen Lösungsmittel, bevorzugt in einem aprotischen Lösungsmittel, beispielsweise Tetrahydrofuran Dioxan, Diethylester umgesetzt. Die Reaktionslösung wird anschließend angesäuert und das Reaktionsprodukt durch Extraktion und Trocknen isoliert.

Die so erhaltenen Verbindungen der allgemeinen Formel II werden nach dem erfindungsgemäßen Verfahren zu den Verbindungen der allgemeinen Formel I reduziert.

Dazu werden die entsprechenden substituierten 2-Cyano-3-hydroxy-3-(phenyl)-acrylsäureester in einem geeigneten Lösungsmittel gelöst, ein Hydrierkatalysator zugegeben und hydriert.

Als Lösungsmittel werden unter Reaktionsbedingungen inerte organische Lösungsmittel, beispielsweise Methanol, Ethanol, Dimethylformamid, Dioxan, Tetrahydrofuran, 1,2 Dimethoxyethan oder Mischungen davon oder Mischungen dieser Lösungsmittel mit Wasser verwendet.
Als Katalysatoren werden Palladium auf Aktivkohle oder Raney-Nickel eingesetzt. Bedeuten X₁ und X₃ in der allgemeinen Formel I beide Chlor, wird bevorzugt Raney-Nickel als Katalysator verwendet.

Die Hydrierung erfolgt je nach Lösungsmittel bei Temperaturen von etwa 0 bis 100°C, vorzugsweise bei Raumtemperatur. Die Hydrierung kann drucklos oder bei einem Druck von etwa 1 bis 5 bar erfolgen, vorzugsweise erfolgt sie drucklos. Die Reaktionsdauer beträgt je nach Substituenten, Reaktionstemperatur, -druck und verwendetem Katalysator etwa 2 - 5 Stunden.

Substituierte 3-Amino-2-(benzoyl)-acrylsäureester stellen wertvolle Zwischenprodukte für antibakterielle Wirkstoffe dar. Die nach dem oben beschriebenen Verfahren erhaltenen Verbindungen können ohne weiteren Reinigungsschritt weiterverarbeitet werden.

Ein weiterer Gegenstand der Erfindung ist daher ein Verfahren zur Herstellung von Zwischenprodukten für antibakterielle Wirkstoffe der allgemeinen Formel III des Formelblattes,
in der X₂, X₃, X₄, X₅, R₁ die in Anspruch 1 angegebene Bedeutung haben, X₁ Halogen, insbesondere Fluor oder Chlor, R₂ H bedeuten oder X₁ und R₂ gemeinsam eine Einfachbindung bilden und R₃ H oder einen gegebenenfalls ein-oder mehrfach substituierten Alkyl-oder Alkoxyrest mit 1 - 6 C-Atomen in der Alkylkette, einen gegebenenfalls substituierten Cycloalkylrest mit 3 - 6 C-Atomen, einen gegebenenfalls ein oder mehrfach substituierten Aryl- oder Heteroarylrest bedeutet, mit der Maßgabe, daß R₂ und R₃ nicht gleichzeitig H bedeuten, dadurch gekennzeichnet, daß man einen substituierten 2-Cyano-3-hydroxy-3-(phenyl)-acrylsäureester der allgemeinen Formel II des Formelblattes,
in der R₁, X₁, X₂, X₃, X₄, X₅ die in Anspruch 1 angegebene Bedeutung haben zu einem substituierten 3-Amino-2(benzoyl)-acrylsäureester der allgemeinen Formel I des Formelblattes,
in der R₁, X₁, X₂, X₃, X₄, X₅ die in Anspruch 1 angegebene Bedeutung haben, selektiv reduziert und anschließend die Verbindungen der allgemeinen Formel I mit einer Verbindung der allgemeinen Formel

R₃NH₂ IV

in der R₃ die oben angegebene Bedeutung hat, gegebenenfalls in Gegenwart einer Säure und in Gegenwart eines Lösungsmittels umsetzt.

Die Umsetzung der substituierten 3-Amino-2(benzoyl)-acrylsäureester erfolgt je nach Nucleophilie des entsprechenden Amins gegebenenfalls in Gegenwart einer Säure, beispielsweise in Gegenwart von Trifluoressigsäure, p-Toluolsulfonsäure oder Essigsäure. Als Lösungsmittel werden beispielsweise Dioxan, Tetrahydrofuran, Acetonitril, Dimethylformamid, 1,2-Dimethoxyethan, Chloroform, Methylchlorid, N-Methylpyrrolidon, Dimethylacetamid verwendet.
Die Reaktion kann bei Temperaturen von etwa 0 - 150°C, vorzugsweise 0 - 80°C erfolgen.

### Beispiel 1:

### 2-Cyano-3-hydroxy-3-(2,4,5-trifluorphenyl)acrylsäuremethylester

26,92 g Natriumhyrid-Dispersion 55 % in Weißöl wurden in 200 ml abs. Tetrahydrofuran suspendiert, auf 5 °C gekühlt und eine Lösung von 32,06 g Cyanessigsäuremethylester in 60 ml abs. THF so zugetropft, daß ein Temperaturintervall von 10 - 15°C eingehalten wurde. Nach beendeter Zugabe wurde 20 min bei ca. 10°C nachgerührt und dann eine Lösung von 60 g 2,4,5-Trifluorbenzolchlorid in 60 ml abs. THF so zugetropft, daß 15 °C nicht überschritten wurden. Anschließend wurde eine weitere Stunde bei ca. 5°C nachgerührt und dann auf 400 ml 4N-HCl/400 g Eis gegossen und das Produkt mit Methylenchlorid extrahiert. Die organische Phase wurde über Natriumsulfat getrocknet und die flüchtigen Bestandteile am Rotationsverdampfer abgezogen. Der kristalline Eindampfrückstand wurde aus Methanol/Essigester umkristallisiert.
75 g (94,2 % d. Th.) farblose Kristalle
Schmp.: 125 - 127 °C (MeOH/Essigester)

### Beispiel 2:

### 2-Cyano-3-(2-chlor-4,5-difluorphenyl)-3-hydroxyacrylsäuremethylester

Die Herstellung erfolgte analog Beispiel 1. Als Ausgangsverbindung wurden 13,91 g 2-Chlor-4,5-difluorbenzoylchlorid verwendet.
14,35 g (79,6 % d. Th.) beige Kristalle
Schmp.: 169 - 71°C (Ethylacetat)

### Beispiel 3:

### 2-Cyano-3-(2,4-dichlor-5-fluorphenyl)-3-hydroxyacrylsäuremethylester

Die Herstellung erfolgte analog Beispiel 1. Als Ausgangsverbindung wurden 15 g 2,4-Dichlor-5-fluorbenzoylchlorid verwendet.
16,47 g (86,1 % d. Th.) beige Kristalle
Schmp.: 168 - 171°C (Essigester)

### Beispiel 4:

### 3-Amino-2-(2,4,5-trifluobenzoyl)-acrylsäuremethylester

29 g des Produktes aus Beispiel 1 wurden in 2000 ml Ethanol gelöst, mit 3 g Palladium 10 % auf Aktivkohle versetzt, entgast und drei Stunden bei Raumtemperatur drucklos hydriert. Der Katalysator wurde abfiltriert, das Filtrat eingedampft und der Rückstand aus Benzol umkristallisiert.
25,11 g (85,3 % d.Th.) hellgrüne Kristalle
Schmp.: 125 - 127 °C (Benzol)

### Beispiel 5:

### 3-Amino-2-(2-chlor-4,5-difluorbenzoyl)acrylsäuremethylester

Die Herstellung erfolgte analog Beispiel 4. Als Ausgangsverbindung wurden 12 g des Produktes aus Beispiel 2 verwendet.
10,5 g (86,9 % d. Th.) beige Kristalle
Schmp.: 139 - 143 °C (i-Propanol) Zersetzung.

### Beispiel 6:

### 3-Amino-2-(2-chlor-4,5-difluorbenzoyl)acrylsäuremethylester

1,0 g des Produktes aus Beispiel 2 wurde in Methanol gelöst, mit 0,5 g Raney-Nickel versetzt und vier Stunden bei Raumtemperatur drucklos hydriert. Der Katalysator wurde abgetrennt, die verbleibende Lösung eingedampft und der Eindampfrückstand aus i-Propanol umkristallisiert.
0,71 g (70,3 % d. Th.) beige Kristalle
Schmp.: 139 - 143 °C (i-Propanol) Zersetzung.

### Beispiel 7:

### 3-Amino-2-(2,4-dichlor-5-fluorbenzoyl)acrylsäuremethylester

Die Herstellung erfolgte analog Beispiel 6. Als Ausgangsverbindung wurden 4 g des Produktes aus Beispiel 3 verwendet.
3,42 g (84,9 % d. Th.) farblose Kristalle
Schmp.: 112 - 115°C (Petroläther)

### Beispiel 8:

### 3-Cyclopropylamino-2-(2-chlor-4,5-difluorbenzoyl)acrylsäure-methylester

2 g des Produktes aus Beispiel 5 und 0,62 g Cyclopropylamin wurden in 30 ml trockenem Acetonitril 30 min bei Raumtemperatur gerührt. Anschließend wurden die flüchtigen Bestandteile am Rotationsverdampfer abgezogen und der Eindampfrückstand aus Äther umkristallisiert.
1,77 g (77,3 % d. Th.) farblose Kristalle
Schmp.: 119 - 121 °C (Diethylether) Zersetzung.

### Beispiel 9:

### 3-Cyclopropylamino-2-(2,4,5-trifluorbenzoyl)acrylsäuremethylester

Zur Herstellung erfolgte analog Beispiel 8. Als Ausgangsverbindung wurde 1 g des Produktes aus Beispiel 4 verwendet.
1,05 g (90,9 % d. Th.) farblose Kristalle
Schmp.: 124 - 127 °C (Ether/Petrolether)

### Beispiel 10:

### 3-((2,4-Difluorphenyl)amino)-2-(2,4,5-trifluorbenzoyl)acrylsäure-methylester

Ein Gemisch bestehend aus 1 g des Produktes von Beispiel 4, 0,88 g (1,2 equ.) p-Toluolsulfonsäure-monohydrat und 1,25 g (2,5 equ.)
2,4-Difluoranilin in 20 ml trockenem Acetonitril wurde eine Stunde zum Rückfluß erhitzt. Nach dem Abkühlen wurde mit Ethylacetat verdünnt, zwei mal mit N-HCl und einmal mit Kochsalzlösung gewaschen, über Natriumsulfat getrocknet, filtriert und eingedampft. Der Eindampfrückstand wurde aus Äther umkristallisiert.
0,95 g (69,8 % d. Th.) farblose Kristalle
Schmp.: 116 - 119°C (Ether)

### Beispiel 11:

### 2-(2-Chlor-4,5-difluorbenzoyl)-3-((4-fluorphenyl)amino)acrylsäuremethylester

Die Herstellung erfolgte analog Beispiel 10 mit 1,0 g des Produktes aus Beispiel 5, 1,01 g p-Fluoranilin und 0,69 g p-Toluolsulfonsäure. H₂O.
0,82 g (61,2 % d. Th.) farblose Kristalle
Schmp.: 98 - 103°C (Diethylether/Petrolether) Zersetzung

### Beispiel 12:

### 6,7-Difluor-1,4-dihydro-4-oxo-chinolin-3-carbonsäuremethylester

5 g des Produktes aus Beispiel 4 wurden in 40 ml Dioxan aufgenommen, mit 0,93 g Natriumhydrid (55 % in Weißöl) versetzt, erst 30 min bei Raumtemperatur gerührt und dann 45 min zum Rückfluß erhitzt. Nach dem Abkühlen wurde auf 150 ml N-HCl gegossen, der anfallende Feststoff abfiltriert und aus Aceton umkristallisiert.
2,91 g (63,1 % d. Th.) hellbeige Kristalle
Schmp.: ab 250°C Sublimation, ab 307 °C Zersetzung (Aceton)

### Beispiel 13:

### 6,7-Difluoro-1,4-dihydro-1-methoxy-4-oxo-chinolin-3-carbonsäuremethylester

Eine Mischung bestehend aus 1,0 g des Produktes aus Beispiel 4 und 0,35 g O-Methylhydroxylamin-Hydrochlorid in 25 ml trockenem Acetonnitril wurde vier Stunden zum Rückfluß erhitzt. Anschließend wurde mit Ethylacetat verdünnt, mit N-HCl und Wasser gewaschen, über Natriumsulfat getrocknet, filtriert und eingedampft. Der kristalline Eindampfrückstand wurde aus Acetonitril umkristallisiert.
0,70 g (67,3 % d. Th.) beige Kristalle
Schmp.: 205 - 208 °C (Acetonitril)

### Beispiel 14:

### 3-Cyclopropylamino-2-(2,4-dichlor-5-fluorbenzoyl)acrylsäuremethylester

Die Herstellung erfolgte analog Beispiel 8. Als Ausgangsverbindung wurde 1,0 g des Produktes aus Beispiel 7 verwendet.
0,72 g (63,3 % d. Th.) farblose Kristallle
Schmp.: 156 - 158°C (Ethylacetat/Äther) Zersetzung.

### Beispiel 15:

### 3-((2,4-Difluorphenyl)amino)-2-(2,4,5-trifluorbenzoyl)acrylsäure-methylester

26,92 g Natriumhyrid-Dispersion 55 % in Weißöl wurden in 200 ml abs. Tetrahydrofuran suspendiert, auf 5 °C gekühlt und eine Lösung von 32,06 g Cyanessigsäuremethylester in 60 ml abs. THF so zugetropft, daß ein Temperaturintervall von 10 - 15°C eingehalten wurde. Nach beendeter Zugabe wurde 20 min bei ca. 10°C nachgerührt und dann eine Lösung von 60 g 2,4,5-Trifluorbenzoylchlorid in 60 ml abs. THF so zugetropft, daß 15 °C nicht überschritten wurden. Anschließend wurde eine weitere Stunde bei ca. 5°C nachgerührt und dann auf 400 ml 4N-HCl/400 g Eis gegossen und das Produkt mit Methylenchlorid extrahiert. Die organische Phase wurde über Natriumsulfat getrocknet und die flüchtigen Bestandteile am Rotationsverdampfer abgezogen.

Der Eindampfrückstand wurde in Methanol gelöst, mit 3 g Palladium 10 % auf Aktivkohle versetzt, entgast und drei Stunden bei Raumtemperatur drucklos hydriert. Der Katalysator wurde abfiltriert und das Filtrat mit 70,22 g p-Toluolsulfonsäuremonohydrat und 100 g 2,4-Difluoranilin versezt und eine Stunde zum Rückfluß erhitzt.
Anschließend wurde am Rotationsverdampfer eingeengt und der Rückstand zwischen 1N-HCl und Ethylacetat verteilt.
Die organische Phase wurde mit Kochsalzlösung gewaschen, über Natriumsulfat getrocknet und eingedampft.
Der Eindampfrückstand wurde aus Ether umkristallisiert.
68,g (60 % d. Th.) farblose Kristalle.
Schmp. 116 - 119°C (Ether)

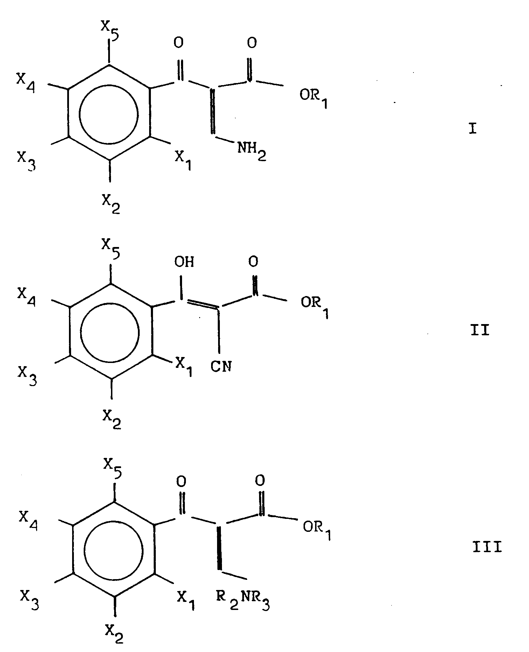

## Patentansprüche

1. Verfahren zur Herstellung von substituierten 3-Amino-2-(benzoyl)-acrylsäureestern der allgemeinen Formel I 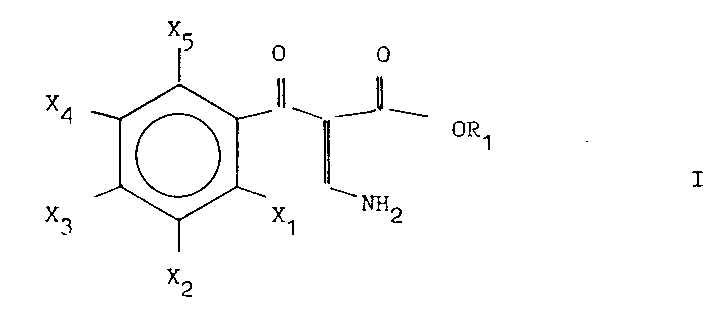 in der R₁ einen Alkylrest mit 1 bis 4 C-Atomen,
X₁ Halogen, insbesondere Fluor oder Chlor,
X₂, X₃, X₄, X₅ Wasserstoff, Halogen, insbesondere Fluor oder Chlor, einen Alkylrest mit 1 - 4 C-Atomen oder einen Alkoxyrest mit 1 - 4 C-Atomen in der Alkylkette bedeuten, dadurch gekennzeichnet, daß man einen substituierten 2-Cyano-3-hydroxy-3(phenyl)-acrylsäureester der allgemeinen Formel II 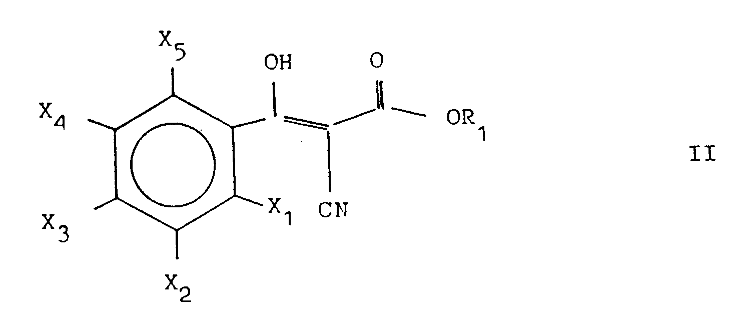 in der R₁, X₁, X₂, X₃, X₄, X₅ die oben angegebene Bedeutung haben selektiv reduziert.

2. Verfahren nach Anspruch 1, dadurch gekennzeichnet, daß die Reduktion in Gegenwart eines Hydrierkatalysators und eines Lösungs- oder Verdünnungsmittels erfolgt.

3. Verfahren nach einem der Ansprüche 1 oder 2, dadurch gekennzeichnet, daß als Hydrierkatalysatoren Palladium auf Aktivkohle oder Raney-Nickel eingesetzt wird.

4. Verfahren nach einem der Ansprüche 1 bis 3, dadurch gekennzeichnet, daß als Lösungs- oder Verdünnungsmittel Methanol, Ethanol, Tetrahydrofuran, Dioxan, Dimethylformamid, 1,2-Dimethoxyethan oder Mischungen davon oder Mischungen mit Wasser eingesetzt werden.

5. Verfahren nach einem der Ansprüche 1 bis 4, dadurch gekennzeichnet, daß die Hydrierung bei Raumtemperatur drucklos erfolgt.

6. Verfahren nach einem der Ansprüche 1 bis 4 zur Herstellung von substituierten 3-Amino-2-(benzoyl)-acrylsäureestern der allgemeinen Formel I nach Anspruch 1 in der R₁ einen Alkylrest mit 1 bis 4 C-Atomen X₁, X₃, X₄ Halogen, insbesondere Fluor oder Chlor und X₂ und X₅ Wasserstoff, Halogen, insbesondere Fluor oder Chlor, einen Alkylrest mit 1 - 4 C-Atomen, oder einen Alkoxyrest mit 1 - 4 C-Atomen in der Alkylkette bedeuten, dadurch gekennzeichnet, daß man einen substituierten 2-Cyano-3-hydroxy-3(phenyl)-acrylsäureester der allgemeinen Formel II, in der R₁, X₁, X₃, X₄, X₂ und X₅ die oben angegebene Bedeutung haben selektiv reduziert.

7. Verfahren zur Herstellung von Zwischenprodukten für antibakterielle Wirkstoffe der allgemeinen Formel III 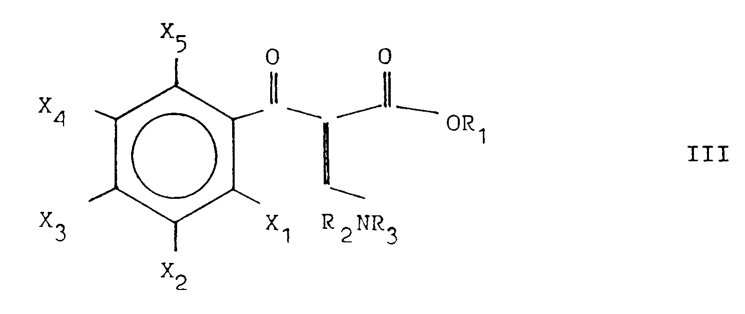 in der X₂, X₃, X₄, X₅, R₁ die in Anspruch 1 angegebene Bedeutung haben, X₁ Halogen, insbesondere Fluor oder Chlor, R₂ H bedeuten oder X₁ und R₂ gemeinsam eine Einfachbindung bilden und R₃ H oder einen gegebenenfalls ein- oder mehrfach substituierten Alkyl- oder Alkoxyrest mit 1 - 6 C-Atomen in der Alkylkette, einen gegebenenfalls substituierten Cycloalkylrest mit 3 - 6 C-Atomen einen gegebenenfalls ein oder mehrfach substituierten Aryl- oder Heteroarylrest bedeutet, mit der Maßgabe, daß R₂ und R₃ nicht gleichzeitig H bedeuten, dadurch gekennzeichnet, daß man einen substituierten 2-Cyano-3-hydroxy-3-(phenyl)-acrylsäureester der allgemeinen Formel II 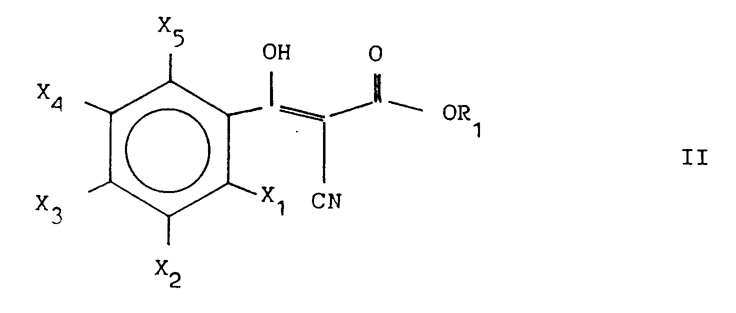 in der R₁, X₁, X₂, X₃, X₄, X₅ die in Anspruch 1 angegebene Bedeutung haben zu einem substituierten 3-Amino-2(benzoyl)-acrylsäureester der allgemeinen Formel I 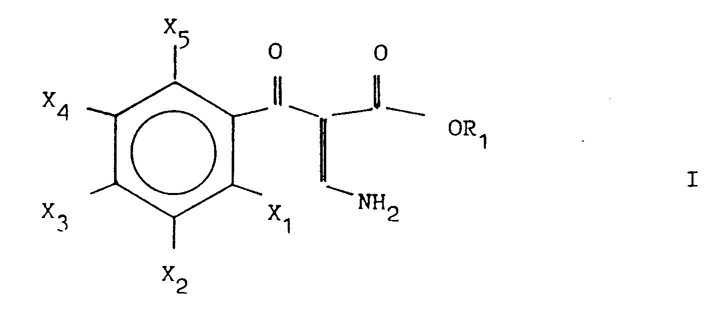 in der R₁, X₁, X₂, X₃, X₄, X₅ die in Anspruch 1 angegebene Bedeutung haben, selektiv reduziert und anschließend die Verbindungen der allgemeinen Formel I mit einer Verbindung der allgemeinen Formel
R₃NH₂ IV
in der R₃ die oben angegebene Bedeutung hat, gegebenenfalls in Gegenwart einer Säure und in Gegenwart eines Lösungsmittels umsetzt.

## Claims

1. Process for the preparation of substituted 3-amino-2-(benzoyl)-acrylic acid esters of the general formula I, 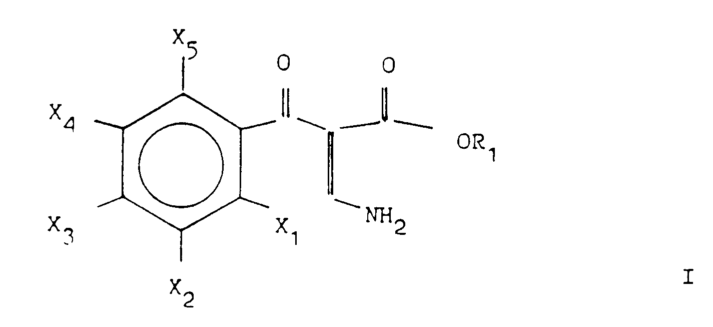 in which R₁ denotes an alkyl radical having 1 to 4 C atoms, X₁ denotes halogen, in particular fluorine or chlorine, X₂, X₃, X₄ and X₅ denote hydrogen, halogen, in particular fluorine or chlorine, an alkyl radical having 1 - 4 C atoms or an alkoxy radical having 1 - 4 C atoms in the alkyl chain, characterized in that a corresponding substituted 2-cyano-3-hydroxy-3(phenyl)-acrylic acid ester of the general formula II, 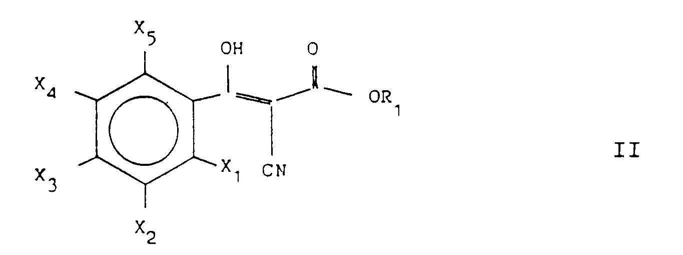 in which R₁, X₁, X₂, X₃, X₄ and X₅ have the abovementioned meaning, are reduced selectively.

2. Process according to Claim 1, characterized in that the reduction is carried out in the presence of a hydrogenation catalyst and a solvent or diluent.

3. Process according to one of Claims 1 or 2, characterized in that palladium-on-active charcoal or Raney nickel is employed as the hydrogenation catalyst.

4. Process according to one of Claims 1 to 3, characterized in that methanol, ethanol, tetrahydrofuran, dioxane, dimethylformamide, 1,2-dimethoxyethane or mixtures thereof or mixtures with water are employed as the solvent or diluent.

5. Process according to one of Claims 1 to 4, characterized in that the hydrogenation is carried out under normal pressure at room temperature.

6. Process according to one of Claims 1 to 4 for the preparation of substituted 3-amino-2-(benzoyl)-acrylic acid esters of the general formula I according to Claim 1, in which R₁ denotes an alkyl radical having 1 to 4 C atoms, X₁, X₃ and X₄ denote halogen, in particular fluorine or chlorine, and X₂ and X₅ denote hydrogen, halogen, in particular fluorine or chlorine, an alkyl radical having 1 - 4 C atoms or an alkoxy radical having 1 - 4 C atoms in the alkyl chain, characterized in that a substituted 2-cyano-3-hydroxy-3-(phenyl)-acrylic acid ester of the general formula II in which R₁, X₁, X₃, X₄, X₂ and X₅ have the meaning given above is reduced selectively.

7. Process for the preparation of intermediate products for antibacterial active compounds of the general formula III, 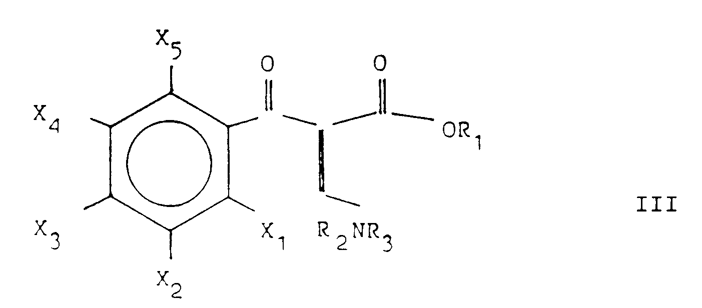 in which X₂, X₃, X₄, X₅ and R₁ have the meaning given in Claim 1, X₁ denotes halogen, in particular fluorine or chlorine, R₂ denotes H, or X₁ and R₂ together form a single bond, and R₃ denotes H or an alkyl or alkoxy radical which has 1 - 6 C atoms in the alkyl chain and is optionally substituted by one or more substituents, an optionally substituted cycloalkyl radical having 3 - 6 C atoms or an aryl or heteroaryl radical which is optionally substituted by one or more substituents, with the proviso that R₂ and R₃ do not simultaneously denote H, characterized in that a substituted 2-cyano-3-hydroxy-3-(phenyl)-acrylic acid ester of the general formula II, 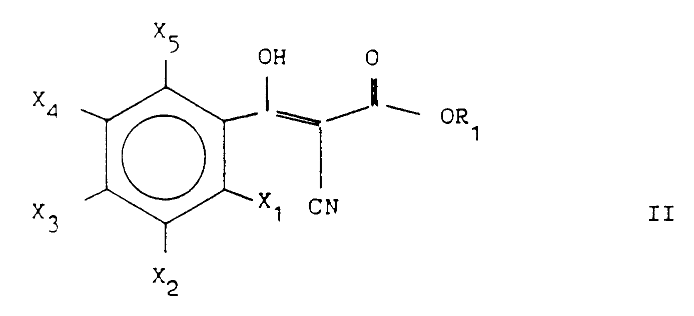 in which R₁, X₁, X₂, X₃, X₄ and X₅ have the meaning given in Claim 1, are reduced selectively to a substituted 3-amino-2(benzoyl)-acrylic acid ester of the general formula I, 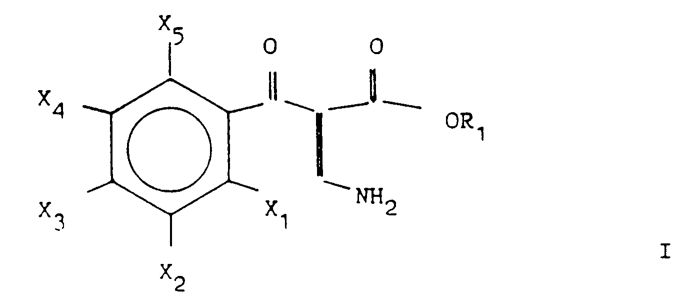 in which R₁, X₁, X₂, X₃, X₄ and X₅ have the meaning given in Claim 1, and the compounds of the general formula I are then reacted with a compound of the general formula
R₃NH₂ IV
in which R₃ has the abovementioned meaning, if appropriate in the presence of an acid and in the presence of a solvent.

## Revendications

1. Procédé de préparation d'esters de l'acide amino-3-(benzoyl)-2-acrylique de formule générale I : 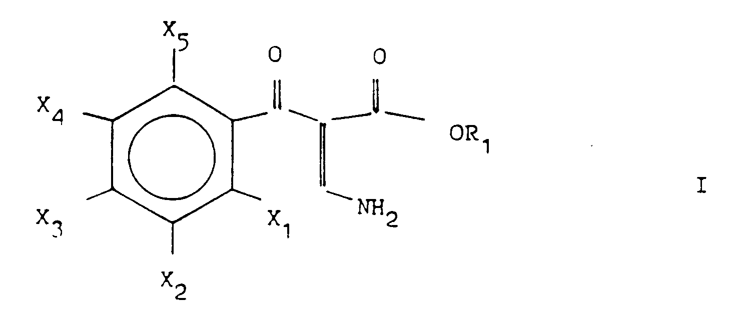 dans laquelle R₁ représente un reste alkyle de 1 à 4 atomes de carbone,
X₁ un halogène, en particulier le chlore ou le fluor,
X₂ , X₃ , X₄ , X₅ un hydrogène, un halogène, en particulier le fluor ou le chlore, un reste alkyle de 1 à 4 atomes de carbone ou un reste alcoxy de 1 à 4 atomes de carbone dans la chaîne alkyle, caractérisé en ce qu'on réduit sélectivement un substitué d'acide cyano-2-hydroxy-3-(phényl)-3-acrylique de formule générale II : 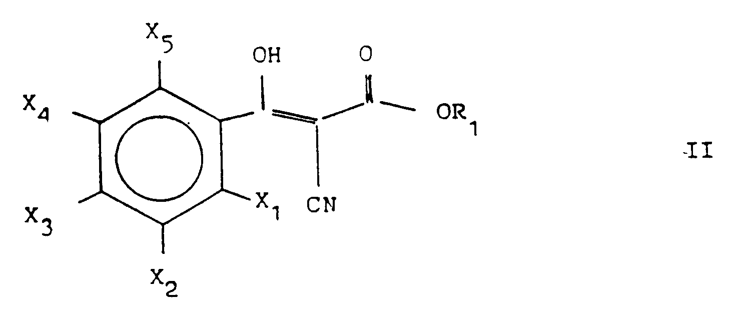 dans laquelle R₁ , X₁ , X₂ , X₃ , X₄, X₅ , ont les significations indiquées ci-dessus.

2. Procédé selon la revendication 1, caractérisé en ce qu'on réalise la réduction en présence d'un catalyseur d'hydrogénation et d'un moyen de solvatation ou de dilution.

3. Procédé selon l'une des revendications 1 ou 2, caractérisé en ce que le catalyseur d'hydrogénation est constitué de palladium sur du charbon actif ou bien du nickel de Raney.

4. Procédé selon l'une des revendications 1 à 3, caractérisé en ce qu'on utilise comme solvant ou diluant le méthanol, l'éthanol, le tétrahydrofurane, le dioxane, le diméthylformamide, le 1,2-diméthoxyéthane ou le mélange de ceux-ci, ou bien leur mélange avec l'eau.

5. Procédé selon l'une des revendications 1 à 4, caractérisé en ce que l'hydrogénation est effectuée à température ambiante et sans pression.

6. Procédé selon l'une des revendications 1 à 4, pour la préparation d'esters substitués de l'acide amino-3-(benzoyl)-2-acrylique de formule générale I selon la revendication 1, dans laquelle R₁ désigne un reste alkyle de 1 à 4 atomes de carbone, X₁ , X₃ X₄ un halogène, en particulier le fluor ou le chlore et X₂ et X₅ l'hydrogène ou un halogène en particulier le fluor ou le chlore, un reste alkyle de 1 à 4 atomes de carbone ou un reste alcoxy de 1 à 4 atomes de carbone dans la chaîne alkyle, caractérisé en ce qu'on réduit de manière sélective un ester substitué de l'acide cyano-2-hydroxy-3-(phényl)-3-acrylique de formule générale II, dans laquelle R₁, X₁, X₃, X₄, X₂ et X₅ ont les significations indiquées ci-dessus.

7. Procédé de préparation de produits intermédiaires pour principes actifs antibactériens de formule générale III : 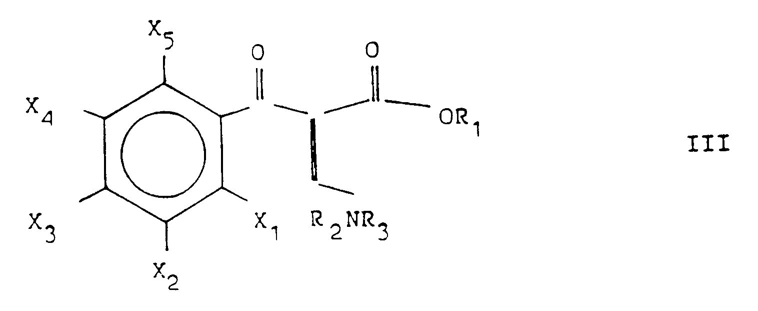 dans laquelle X₂ , X₃ , X₄ , X₅ , R₁ ont la signification donnée dans la revendication 1, X₁ représente un halogène en particulier le fluor ou le chlore, R₂ représente H, ou X₁ et R₂ constituent ensemble une simple liaison, R₃ représente H ou bien un reste alkyle ou alcoxy le cas échéant, mono- ou poly-substitué, de 1 à 6 atomes de carbone dans la chaîne alkyle, un reste cycloalkyle substitué le cas échéant ayant 3 à 6 atomes de carbone, un reste aryle ou hétéroaryle mono-ou poly-substitué à l'exception du fait que R₂ et R₃ ne représentent pas simultanément l'hydrogène, caractérisé en ce qu'on réduit sélectivement un ester substitué de l'acide cyano-2-hydroxy-3-(phényl)-3-acrylique de formule générale II : 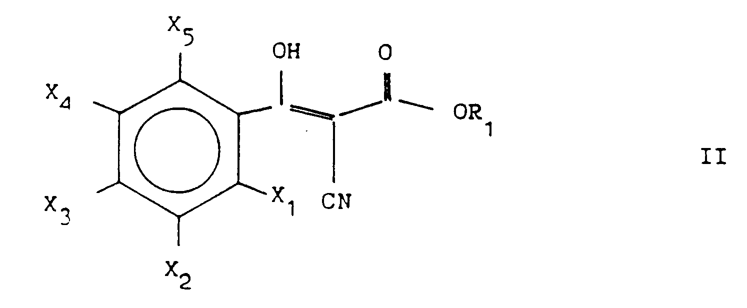 dans laquelle R₁ , X₁ , X₂ , X₃ , X₄ , X₅ ont la signification indiquée dans la revendication 1, en un ester substitué d'acide amino-3-(benzoyl)-2-acrylique de formule générale I : 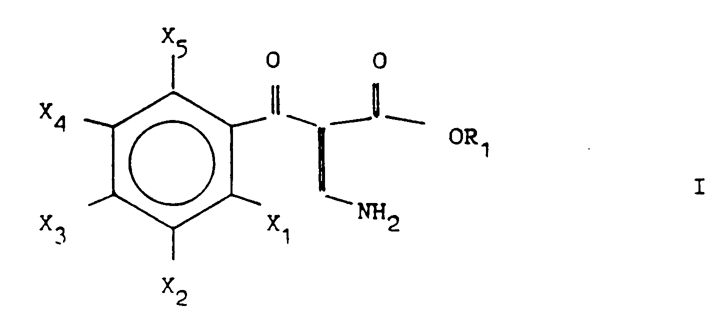 dans laquelle R₁ , X₁ , X₂ , X₃ , X₄ , X₅ ont la signification donnée dans la revendication 1, puis on fait réagir les composés de formule générale I avec un composé de formule générale IV :
R₃NH₂ IV
dans laquelle R₃ a la signification indiquée ci-dessus, le cas échéant en présence d'un acide et en présence d'un solvant.
